# EUROPÄISCHE PATENTANMELDUNG

(11) **EP 1 818 040 A1**
(43) Veröffentlichungstag der Anmeldung: **15.08.2007**
(21) Anmeldenummer: 07002461.7
(22) Anmeldetag: 05.02.2007
(51) Int. Cl.: A61K 6/10, A61C 13/00

(54) **Verfahren zur Herstellung von Zahnrestaurationen bzw. Zahnersatzteilen**

(30) Priorität: 09.02.2006 DE 102006006081
(71) Anmelder: Kaltenbach & Voigt GmbH, 88400 Biberach (DE)
(72) Erfinder: Bodenmiller, Anton, 88299 Leutkirchen (DE); Paranjape, Amit, 88339 Waldsee (DE)
(74) Vertreter: Thun, Clemens

(57) **Zusammenfassung**

Bei einem Verfahren zur Herstellung von Zahnrestaurationen bzw. Zahnersatzteilen aus einem nichtmetallischen, anorganischen Material wird zunächst ein modellierfähiger Werkstoff in plastischer Form im Mund eines Patienten oder unter Nutzung eines Modells zu einem Formteil modelliert (103; 205). Anschließend wird das Formteil aus dem Mund des Patienten entnommen bzw. von dem Modell entfernt (105; 208) und zu einer keramischen Zahnrestauration bzw. einem Zahnersatzteil ausgehärtet (106; 209).

## Beschreibung

Die vorliegende Erfindung betrifft ein Verfahren zur Herstellung von Zahnrestaurationen bzw. Zahnersatzteilen aus einem biokompatiblen Material.

Die Herstellung von Zahnersatzteilen nimmt nach wie vor eine zentrale Rolle in der Dentalmedizin ein. Erste individuelle Porzellanzähne wurden bereits im Jahr 1808 in Frankreich hergestellt. Um 1900 erfolgte dann die Entwicklung der so genannten Keramik-Jacket-Krone, welche die Grundlage für die heutige Metall-Verblendkeramik bildet. Solid-Keramik für Einzelkronen, Inlays, Onlays und Veneers wurden dann ab 1982 in der Zahntechnik als metallfreie Alternativen eingesetzt.

Die wichtigsten Kriterien für den Einsatz neuer Werkstoffe zur Herstellung von Zahnrestaurationen bzw. Zahnersatzteilen sind nach wie vor eine geeignete Biokompatibilität sowie die mit dem Material erzielbare Ästhetik. Mit Einführung der modernen Hochleistungs-Oxidkeramiken und speziell vergüteter Glaskeramiken steht der Zahnmedizin heute ein breites Werkstoffspektrum zur Verfügung, um den Patienten mit biokompatiblem, ästhetischem und auch dauerhaft haltendem keramischen Zahnersatz zu versorgen. Voraussetzung hierfür ist allerdings ein konsequent durchgehender, auf den keramischen Werkstoff genauestens abgestimmter Prozess. Dieser beginnt mit einer keramikgerechten Präparation des Zahns, einer auf das Keramikmaterial abgestimmten Konstruktion, der Fertigung und Verblendung im Dentallabor sowie einer der Situation des Patienten entsprechenden Befestigung mit Kompositen oder Zementierung im Patientenmund.

Je nach Indikation kommen dabei heutzutage unterschiedlichste Keramiksysteme zum Einsatz. Zur Herstellung von Inlays, Onlays, Veneers und Frontzahnkronen werden beispielsweise leuzitverstärkte Glaskeramiken als Presskeramiken eingesetzt. Ferner werden derartige leuzitverstärkte Glaskeramiken auch als Schleifkeramiken unter der Nutzung von CAD-/CAM-Systemen verwendet. Ein hierzu alternatives System bildet die Nutzung von Gerüstkappen bestehend aus Al₂O₃ oder ZrO₂ sowie einer geeigneten Verblendung. Diese Kombination wird beispielsweise zur Herstellung von Frontzahnkronen, Seitenzahnkronen oder Molarenkronen verwendet. Verwandt hierzu sind Brückengerüste aus ZrO₂ mit geeigneter Verblendung, welche für die Herstellung von Brücken im Frontzahnbereich, Brücken im Seitenzahnbereich sowie weitspannige Molarenbrücken eingesetzt werden. Schließlich werden ZrO₂-Keramiken mit einer entsprechenden Verblendung auch zur Herstellung so genannter Implantat-Abutments oder von Implantataufbauten verwendet.

In letzter Zeit stehen vor allem für Molarenkronen auch völlig neuartige Werkstoffe zur Verfügung, welche es ermöglichen, Kronen inklusive Kaufläche aus Keramiken ohne zusätzliche Verblendung herzustellen. Dies wird dadurch ermöglicht, dass das hierfür eingesetzte Material, bei dem es sich z.B. um eine Zirkon-/Silikatkeramik handelt, während des Sinterns nicht schrumpft.

In jedem Fall erfordern keramische Inlays, Onlays, Veneers, Frontzahnkronen, Seitenzahnkronen und Molarenkronen als hoch-biokompatible Patientenversorgung eine aufwendige und teuere technische Fertigung unter Einbeziehung von Spezialisten. Dies ist unabhängig davon, ob die Zahnersatzteile in der Zahnarztpraxis selbst hergestellt, vom Dentallabor gefertigt oder bei einem industriellen Massenfertiger produziert werden. Beim Zahnarzt werden hierzu vorzugsweise CAD-/CAM-Schleifsysteme in Verbindung mit einer Intraoral-Kamera eingesetzt. Im Dentallabor wiederum steht die Fertigung mit Press-Keramiken ähnlich der Metallgusstechnik im Vordergrund. Auch CAD-/CAM-basierte Fräs-/Schleiffertigung und die so genannte Schlickerfertigung werden im Dentallabor eingesetzt. Vergleichbare Herstellungsverfahren kommen schließlich auch beim industriellen Massenfertiger zum Einsatz, wobei allerdings bei der Nutzung von Press-Keramiken die Fertigung hoch automatisiert vorgenommen wird. Auch bei der Nutzung anderer Techniken werden vorzugsweise Halb- oder Vollautomaten eingesetzt.

Hoch-biokompatibler Zahnersatz, wie er aus medizinischen Gründen grundsätzlich wünschenswert wäre, wird derzeit nach wie vor auf die zuvor beschriebene Weise hergestellt. Bedingt durch die Fertigungstechnologie, die hiermit verbundenen Fertigungszeiten und den Werkstoffeinsatz ist allerdings ein vollständig biokompatibler, keramischer Zahnersatz für den Patienten nach wir vor sehr teuer, weshalb die Nutzung derartiger Keramik-Ersatzteile noch nicht zur Standardversorgung zählt. Stattdessen werden für die Hauptanwendungsgebiete der Zahnmedizin, nämlich die Füllung von Zahndefekten, heutzutage mit Amalgam - was allerdings nur noch selten angewandt wird - und Kunststoffen zwar preisgünstige Materialien verwendet, welche allerdings nicht in hohem Maße biokompatibel und darüber hinaus auch in ihrer Haltbarkeit beschränkt sind.

Im Hinblick auf die extrem hohe Anzahl der jährlich anfallenden Versorgungen von Patienten weltweit liegt dementsprechend der vorliegenden Erfindung die Aufgabe zu Grunde, ein Verfahren und einen Werkstoff zur Verfügung zu stellen, welches bzw. welcher die kostengünstige Herstellung eines hochqualitativen Zahnersatzteils ermöglicht.

Die Aufgabe wird durch ein Verfahren gemäß Anspruch 1 sowie durch ein nichtmetallisches, anorganisches Material zur Herstellung von Zahnrestaurationen bzw. Zahnersatzteilen gemäß Anspruch 9 gelöst. Vorteilhafte Weiterbildungen der Erfindung sind Gegenstand der Unteransprüche.

Die erfindungsgemäße Lösung beruht auf der Idee, eine modellierbare, nichtmetallisch-anorganische Masse zu verwenden, welche es erlaubt, im plastifizierten Zustand in die Kavität oder andere Versorgungen eingebracht zu werden. Im Mund des Patienten oder alternativ hierzu auch im Modell beim Zahntechniker kann dann dieses Material mit üblichen Instrumentarien modelliert, d. h. an die okklusale Gegebenheit angepasst und damit zu einem Formteil mit geeigneten Konturen verformt werden. Nach dieser Formgebung kann dann das modellierte Teil aus dem Patientenmund oder dem Modell wieder entfernt werden, um es anschließend in einem thermischen Prozess zu einer biokompatiblen, keramischen, prothetischen Versorgung auszuhärten. Hierzu ist vorzugsweise vorgesehen, dass bereits vor der Entnahme des Teils aus dem Patientenmund bzw. dem Modell dieses vorgehärtet wird, um sicherzustellen, dass das Teil bei der Entnahme nicht deformiert wird. Auf diese Weise wird eine einfache Herstellung einer Zahnrestauration bzw. eines Zahnersatzteils ermöglicht, welches sowohl hinsichtlich seiner biokompatiblen Eigenschaften als auch hinsichtlich seiner Formgenauigkeit höchsten Anforderungen genügt.

Erfindungsgemäß wird also ein Verfahren zur Herstellung von Zahnrestaurationen bzw. Zahnersatzteilen aus einem nichtmetallischen, anorganischen Material vorgeschlagen, wobei zunächst ein modellierfähiger Werkstoff in plastischer Form im Mund eines Patienten oder unter Nutzung eines Modells zu einem Formteil modelliert wird, anschließend das Formteil aus dem Mund des Patienten entnommen bzw. von dem Modell entfernt wird und abschließend zu einem keramischen Ersatzteil ausgehärtet wird. Vorzugsweise wird das modellierte Formteil vor der Entnahme aus dem Mund des Patienten bzw. dem Entfernen von dem Modell vorgehärtet.

Für das erfindungsgemäße Verfahren ist es von Vorteil, Werkstoffe zu nutzen, wie sie neuerdings für die Fertigung in CAD-/CAM-Systemen bekannt sind. Insbesondere ist der Einsatz von Zirkon/Silikatkeramiken sinnvoll, welche die Eigenschaft haben, nur sehr gering bzw. gar nicht während des thermischen Behandlungsprozesses zu schrumpfen. Diese Materialien wurden bislang überwiegend in einem Festzustand bearbeitet, wozu aufwendige und teuere Maschinen erforderlich waren. Bei der Nutzung einer modellierfähigen Masse gemäß dem erfindungsgemäßen Verfahren hingegen ist es möglich, die Gestaltung der Zahnrestauration bzw. des Zahnersatzteils durch übliche, einfache oder manuelle Modellationen vorzunehmen, weshalb abschließend nur noch ein geeignetes, beispielsweise thermisches Verfahren zur Fertigstellung des keramischen Zahnersatzes erforderlich ist.

Je nach Indikation, beispielsweise Inlay oder Krone, kann es sinnvoll sein, das Schrumpfverhalten des eingesetzten erfindungsgemäßen Werkstoffs anzupassen. Beispielsweise ist bei der Herstellung von Inlays, welche adhäsiv eingesetzt werden, eine Schrumpffreiheit wünschenswert, während hingegen beim Einzementieren von Kronen ein gewisser Volumenschrumpf durchaus von Vorteil ist, um den Zementspalt berücksichtigen zu können. Diese gewünschte Schrumpfung kann bei dem erfindungsgemäßen Verfahren durch Nutzung eines geeigneten Werkstoffs berücksichtigt werden.

Das Vorhärten der plastifizierten Masse im Patientenmund oder im Modell kann durch unterschiedliche Maßnahmen erfolgen. Beispielsweise wäre die Zugabe von Bindemitteln, die ähnlich den heutigen Kunststofffüllungsmaterialien durch Licht härtbar sind, denkbar. Auch der Einsatz von Mehrkomponentensystemen, welche zeitabhängig zunehmend härter werden und zum Zeitpunkt der Entnahme des Formteils eine ausreichende Stabilität aufweisen, wäre möglich. Schließlich könnten auch andere technische Lösungen, wie Laserhärten, chemische Behandlung oder ähnliche Verfahren eingesetzt werden.

Das Aushärten bzw. Sintern des Formteils außerhalb des Patientenmundes bzw. des Modells kann dann in geeigneter Weise analog dem heutigen Sinterverfahren bei keramischen Werkstoffen in einem Sinterofen erfolgen. Bei Nutzung eines geeigneten Materials für das Modell wäre im Übrigen auch das Aushärten im Modell selbst denkbar. Je nach Materialspezifikation ist jedoch auch die Verwendung eines handelsüblichen keramischen Brennofens denkbar. Auch der Einsatz anderer Verfahren, zum Beispiel dem Einsatz von Mikrowellen oder Laserstrahlung zum Aushärten, wäre möglich.

Der Vorteil des erfindungsgemäßen Verfahrens liegt zum einen in einer bislang nicht bekannten hohen Wirtschaftlichkeit bei der Patientenversorgung mit hoch-biokompatiblem Zahnersatz und Füllungen. Ein weiterer Vorteil liegt in der nahtlosen Übernahme bestehender Praktiken beim Zahnarzt für Füllungen bzw. der Modellationstechnik beim Zahntechniker. Insbesondere sind keine besonderen Schulungen erforderlich, da die Arbeitsweise des Zahnarztes keine prinzipielle Neuorientierung erfordert. Ferner beruht die hohe Wirtschaftlichkeit des erfindungsgemäßen Verfahrens auch darauf, dass keine aufwendige Geräteinvestition erforderlich ist. Ein Großteil der bisher separat von Spezialisten durchgeführten Arbeiten, wie Aufmodellieren, gnathologische Anpassung mittels Artikulatoren und dergleichen, kann nämlich entfallen.

Nachfolgend soll das erfindungsgemäße Verfahren zur Herstellung von Zahnrestaurationen bzw. Zahnersatzteilen anhand der beiliegenden Zeichnung näher erläutert werden. Es zeigen:
- Fig. 1: die Schritte eines ersten erfindungsgemäßen Verfahrens zur Herstellung eines Zahnersatzteils und
- Fig. 2: die Schritte eines hierzu alternativen Herstellungsverfahrens gemäß der vorliegenden Erfindung.

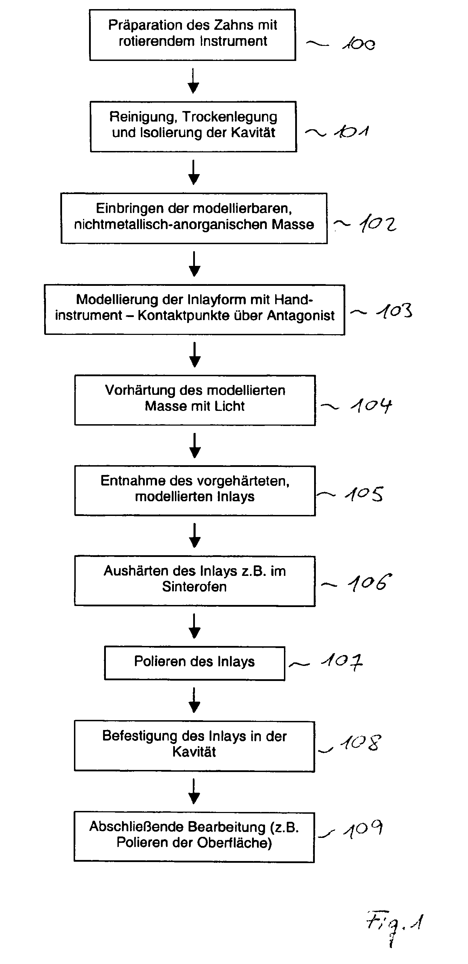

Figur 1 zeigt schematisch die einzelnen Schritte bei der Durchführung eines ersten Ausführungsbeispiels des erfindungsgemäßen Verfahrens zur Herstellung eines Zahnersatzteils. Wie bereits zuvor geschildert wurde, basiert die erfindungsgemäße Lösung grundsätzlich auf dem Gedanken, ein nichtmetallisches, anorganisches Material einzusetzen, welches als modellierfähiger Werkstoff in plastischer Form zur Verfügung gestellt wird, der einerseits die einfache Herstellung eines noch nicht vollständig ausgehärteten Formteils ermöglicht, andererseits - gegebenenfalls nach einem entsprechenden Vorhärten - eine ausreichende Stabilität aufweist, um das Formteil vor dem endgültigen Aushärten aus dem Patientenmund bzw. von einem Modell entfernen zu können. Das hierzu genutzte Material weist dementsprechend einerseits eine Keramik-Komponente auf, welche das abschließende Aushärten zu einem hochfesten Keramikteil ermöglicht. Andererseits ist ein Bindemittel vorhanden, welches zunächst das Keramik-Rohmaterial zu einer plastischen Masse bindet, weiterhin ein Vorhärten ermöglicht, um die Entnahme des modellierten Formteils zu gewährleisten, und welches sich abschließend beim Sintern des Formteils verflüchtigt.

Das Verfahren zur Herstellung eines Zahnersatzteils in Form eines Inlays gemäß der ersten Variante läuft dann gemäß dem folgenden Schema ab.

In einem ersten Schritt 100 wird zunächst der Zahn, der mit einem Zahnersatzteil versehen werden soll, mit einem rotierenden Instrument versorgt. Auch die Nutzung anderer abhebender Verfahren zur Präparation des Zahns wäre selbstverständlich denkbar. In dem darauf folgenden Schritt 101 wird dann die Kavität gereinigt, trocken gelegt und isoliert, so dass sie zur Herstellung des Zahnersatzteils genutzt werden kann.

Erster zentraler Punkt des erfindungsgemäßen Verfahrens ist dann Schritt 102, in dem die modellierbare, nichtmetallisch-anorganische Masse in die Kavität eingebracht wird. In dem Patientenmund wird dann in dem Schritt 103 mittels Handinstrumenten in gewohnter Weise die Inlayform aus der plastischen Masse modelliert, wobei die Kontaktpunkte der Inlayform über den Antagonisten des mit dem Zahnersatzteil zu versorgenden Zahns erhalten werden. Das Modellieren des Formteils erfolgt also in gleicher Weise, wie beim Zahnarzt üblicherweise Zahnfüllungen, beispielsweise aus Kunststoffmaterial, geformt werden.

Nach Beendigung der Modellierung der Inlayform wird dann in Schritt 104 das modellierte Formteil vorgehärtet, was insbesondere durch die Bestrahlung des Materials mittels Licht erfolgen kann. Auch die Nutzung anderer Systeme, welche es ermöglichen, der Masse eine gewisse Stabilität zu verleihen, wäre denkbar. Insbesondere könnte auch der Einsatz von Mehrkomponentensystemen genutzt werden, bei denen die Masse mit zunehmender Zeit eine höhere Stabilität aufweist.

Das auf diese Weise vorgehärtete, modellierte Inlay wird dann im Schritt 105 aus dem Patientenmund entnommen, wobei die Stabilität des Materials nunmehr so hoch ist, dass das Formteil bei der Entnahme nicht beschädigt bzw. verformt wird. Im nachfolgenden Schritt 106 wird dann das Formteil in klassischer Weise ausgehärtet, was beispielsweise in einem Sinterofen erfolgt. Auf diese Weise wird ein hochfestes Keramik-Ersatzteil geformt, welches anschließend dann in Schritt 107 poliert wird. Schließlich wird das Inlay in der Kavität des zu behandelnden Zahns befestigt (Schritt 108). Als abschließende Bearbeitung kann in Schritt 109 beispielsweise ein Polieren der Oberfläche des Ersatzteils vorgesehen sein.

Aus der zuvor erfolgten Schilderung des erfindungsgemäßen Verfahrens ergibt sich, dass die Herstellung des Zahnersatzteils in sehr einfacher Weise unmittelbar durch den Zahnarzt durchgeführt werden kann. Insbesondere ist nicht der Einsatz aufwendiger Maschinen zur Bearbeitung des Ersatzteils erforderlich, weshalb das Verfahren äußerst kostengünstig durchzuführen ist. Wird ferner ein Material eingesetzt, welches im abschließenden Sinterschritt 106 nahezu keine Schrumpfung aufweist, kann sichergestellt werden, dass das Ersatzteil eine sehr hohe Passgenauigkeit für die Zahnkavität aufweist.

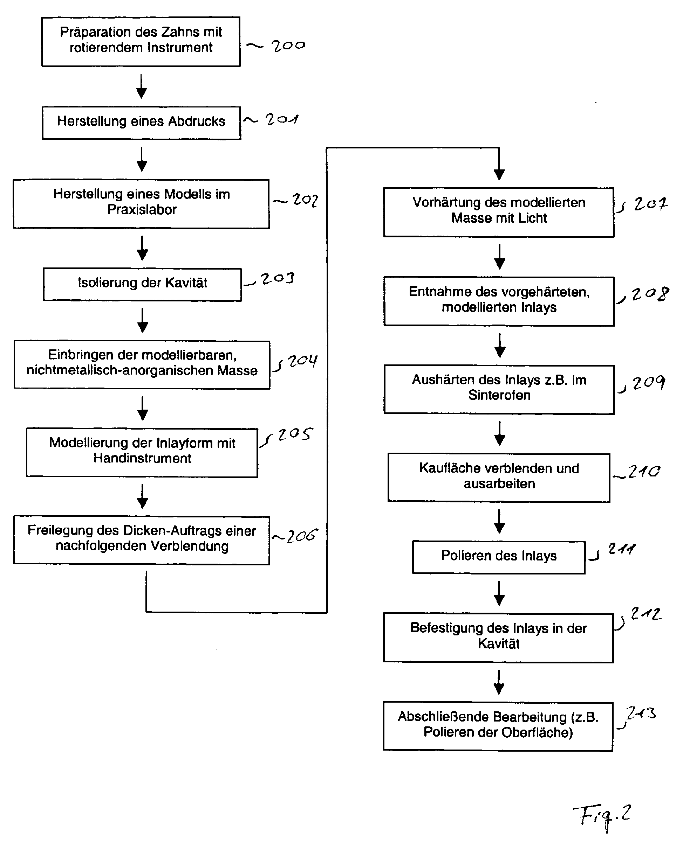

Alternativ zu dem soeben geschilderten Verfahren bestünde auch die Möglichkeit, das Zahnersatzteil beim Zahntechniker herzustellen. Hierzu kommt ein modifiziertes Verfahren zum Einsatz, dessen Schritte in Figur 2 dargestellt sind.

Das alternative Verfahren beginnt wiederum zunächst mit dem Schritt 200, in dem der Zahn mit einem rotierenden Instrument oder anderweitig abhebend präpariert wird. Nachfolgend wird nunmehr allerdings ein Abdruck genommen (Schritt 201), welcher im darauf folgenden Schritt 202 die Herstellung eines Modells im Praxislabor ermöglicht. Anhand dieses Modells wird wiederum die Kavität isoliert (Schritt 203), in welche dann - wie bei dem vorherigen Verfahren gemäß Fig. 1 - eine modellierbare, nichtmetallisch-anorganische Masse eingebracht wird (Schritt 204).

Ebenso wie bei dem vorherigen Verfahren wird anschließend mit Hilfe eines Handinstruments in Schritt 205 die Inlayform modelliert, wobei in Schritt 206 insbesondere auch die Freilegung des Dicken-Auftrags einer nachfolgenden Verblendung berücksichtigt werden kann. Nachfolgend wird die modellierte Masse wiederum vorgehärtet (Schritt 207) und in Schritt 208 aus dem Modell entnommen. Um diese Entnahme zu erleichtern, kann - auch bei dem Verfahren gemäß Figur 1 - das Formteil beispielsweise mit einem Hilfselement in Form eines Stifts, Vorsprungs oder Zapfens versehen werden, welches zu einem späteren Zeitpunkt wieder entfernt wird. Auch die Entnahme mit Hilfe eines Werkzeugs - z. B. Gewindebohrers - wäre denkbar, wobei das hierbei eventuell gebildete Loch dann vor dem abschließenden Aushärten des Formteils wieder gefüllt wird.

Zur Fertigstellung des Keramikersatzteils wird das modellierte Inlay dann in Schritt 209 ausgehärtet, wobei bei Nutzung eines geeigneten Modellmaterials auch denkbar wäre, das Zahnersatzteil unmittelbar in dem Modell auszuhärten. In Schritt 210 schließlich wird die Kaufläche verblendet und ausgearbeitet, woran sich der Vorgang des Polierens des Inlays (Schritt 211) anschließt. Schließlich wird das Inlay in Schritt 212 wiederum in der Kavität des Zahns des Patienten befestigt. Hieran kann sich eine abschließende Bearbeitung (Schritt 213) anschließen.

Dieses alternative Verfahren ermöglicht es somit, zusätzliche Verblendungen einzusetzen. Zwar ist wiederum die Herstellung in einem Zahnlabor erforderlich, allerdings ist der Aufwand zur Bearbeitung des Materials im Vergleich zu bislang eingesetzten Materialien deutlich geringer.

Analog zu der oben ausgeführten beispielhaften Inlayfertigung lassen sich in ähnlicher Weise mit der erfindungsgemäßen modellierbaren, nichtmetallisch-anorganischen Masse beim Zahnarzt, im Praxislabor, im Dentallabor auch Einzelkronen für den Seitenzahn- und Molarenbereich herstellen. Auch größere Arbeiten wären denkbar.

Insgesamt gesehen wird somit durch die vorliegende Erfindung die Möglichkeit geschaffen, hoch-kompatible und passgenaue Zahnersatzteile in einfacher und insbesondere wirtschaftlicher Weise zu fertigen. Auf den Einsatz teuerer Maschinen zur Bearbeitung des Materials kann dabei verzichtet werden. Für den Zahnarzt eröffnet sich auf diese Weise die Möglichkeit, Zahnersatzteile, welche höchsten Ansprüchen genügen, in einfacher Weise herzustellen.

## Patentansprüche

1. Verfahren zur Herstellung von Zahnrestaurationen bzw. Zahnersatzteilen aus einem nichtmetallischen, anorganischen Material, wobei
a) ein modellierfähiger Werkstoff in plastischer Form im Mund eines Patienten oder unter Nutzung eines Modells zu einem Formteil modelliert wird (103; 205),
b) das Formteil aus dem Mund des Patienten entnommen bzw. von dem Modell entfernt wird (105; 208) und
c) das Formteil zu einer keramischen Zahnrestaurationen bzw. einem Zahnersatzteil ausgehärtet wird (106; 209).

2. Verfahren nach Anspruch 1,
**dadurch gekennzeichnet,**
**dass** das modellierte Formteil vor der Entnahme aus dem Mund des Patienten bzw. dem Entfernen von dem Modell vorgehärtet wird (104; 207).

3. Verfahren nach Anspruch 2,
**dadurch gekennzeichnet,**
**dass** das Vorhärten (106; 207) durch Bestrahlung des Formteils erfolgt.

4. Verfahren nach Anspruch 2,
**dadurch gekennzeichnet,**
**dass** der Werkstoff eine Komponente enthält, durch deren Wirkung der Werkstoff mit zunehmender Zeit eine höhere Stabilität aufweist.

5. Verfahren nach einem der vorherigen Ansprüche,
**dadurch gekennzeichnet,**
**dass** das der modellierfähige Werkstoff während des Aushärtens (106; 209) nicht schrumpft.

6. Verfahren nach einem der Ansprüche 1 bis 4,
**dadurch gekennzeichnet,**
**dass** der modellierfähige Werkstoff während des Aushärtens (106; 209) um ein vorgesehenes Maß schrumpft.

7. Verfahren nach einem der vorherigen Ansprüche,
**dadurch gekennzeichnet,**
**dass** die Entnahme (105; 208) des Formteils aus dem Mund des Patienten bzw. dem Modell mit Hilfe eines Hilfselements erfolgt.

8. Verfahren nach Anspruch 7,
**dadurch gekennzeichnet,**
**dass** das Hilfselement ein an dem Formteil vorgesehener Stift bzw. ein Vorsprung ist.

9. Material zur Herstellung von Zahnrestaurationen bzw. Zahnersatzteilen aufweisend
• eine Keramik-Komponente, welche ein Aushärten des Materials zu einem Keramikteil ermöglicht,
• ein Bindemittel, welches das Material in einen plastifizierten Zustand versetzt.

10. Material nach Anspruch 9,
**dadurch gekennzeichnet,**
**dass** das Bindemittel ein Vorhärten eines aus dem Material gebildeten Formteils ermöglicht.
